# EUROPEAN PATENT APPLICATION

(11) **EP 2 027 850 A1**
(43) Date of publication of application: **25.02.2009**
(21) Application number: 07114763.1
(22) Date of filing: 22.08.2007
(51) Int. Cl.: A61K 9/08, A61K 31/506, A61K 47/22, A61P 31/10

(54) **Pharmaceutical compositions containing voriconazole**

(71) Applicant: SANDOZ AG, 4056 Basel (CH)
(72) Inventor: Welz, Christian, 6230 Brixlegg (AT); Jennewein, Herwig, 6067 Absam (AT); Raneburger, Johannes, 6300 Wörgl (AT); Schmarda, Andreas, 6091 Goetzens (AT)
(74) Representative: Kröger, Bernd

(57) **Abstract**

The present invention provides a process for improving the solubility of voriconazole in aqueous solutions. The present invention also provides a composition comprising voriconazole and N-methylpyrrolidone obtainable by this process, a method of treating a fungal infection, a pharmaceutical composition comprising voriconazole and N-methylpyrrolidone and the use of a therapeutically effective amount of the composition comprising voriconazole and N-methylpyrrolidone and at least one pharmaceutically acceptable excipient for the preparation of a medicament for treating a fungal infection.

## Description

### Field of the Invention

The present invention relates to a process for improving the solubility of voriconazole in aqueous solutions, and compositions comprising voriconazole and N-methylpyrrolidone, which may be used in pharmaceutical compositions.

### Background of the Invention

(2R,3S)-2-(2,4-Difluorophenyl)-3-(5-fluoro-4-pyrimidinyl)-1-(1H-1,2,4-triazol-1-yl)-butan-2-ol, also known as voriconazole, is disclosed in EP-A 440 372. Voriconazole is useful in the treatment of fungal infections. Voriconazole has a low aqueous solubility (0.61 mg/ml at a pH 7; 0.2 mg/ml at a pH 3), and is not stable in water. Thus, development of an aqueous intravenous formulation with a sufficient shelf life is difficult. These problems are magnified by the semi-polar nature of the compound (log D=1.8) which means that it is not generally solubilised by conventional means such as oils, surfactants or water miscible co-solvents.

For solid drugs it is also important to render the sparingly water-soluble drug water-soluble since a good solubility increases the bioavailability of the drug. It has been described that inclusion compounds, e.g. with urea or complexes of polyvinyl pyrrolidone may improve the solubility of a compound but in aqueous solution they are not stable. Such inclusion compounds are therefore at best suitable for solid application forms of drugs.

WO 98/58677 discloses that the solubility of voriconazole in water can be increased by molecular encapsulation with sulphoalkylether cyclodextrin derivatives of the type disclosed in WO 91/11172, particularly beta-cyclodextrin derivatives wherein the cyclodextrin ring is substituted by sulphobutyl groups. There are complex manufacturing issues associated with cyclodextrin formulations that significantly increase cost of goods.

WO 97/28169 discloses a phosphate pro-drug of voriconazole, which exhibits increased solubility and aqueous stability. However, the pro-drug does not exhibit 100% bioequivalence to voriconazole, such that cost of goods is again significantly increased.

US 2005/112204 relates to a new pharmaceutical formulation of voriconazole, in particular a formulation comprising one or more poloxamers.

US 6,632,803 provides pharmaceutical formulation comprising voriconazole, or a pharmaceutically acceptable derivative thereof, and a sulfobutylether beta-cyclodextrin.

WO 2004/032902 relates to compositions of an aqueous suspension of submicron- to micron-size particles of an antifungal agent coated with one or more surfactants. The particles of the antifungal agent should have a volume-weighted mean particle size of less than about 50µm in diameter. In one embodiment, the antifungal agent is a triazole antifungal agent such as itraconazole, ketoconazole, miconazole, fluconazole, ravuconazole, voriconazole, saperconazole, eberconazole, genaconazole, and posaconazole.

US 5,773,443 relates to triazole derivatives which have antifungal activity, more particularly to 2-aryl-3-(3-halopyridin-4-yl or 5-halopyrimidin-4-yl)-1-(1H-1,2,4-triazol-1-yl)alkan-2-ol derivatives which are useful in the treatment of fungal infections in animals, including human beings.

US 5,116,844 relates to novel triazole derivatives which have antifungal activity and are useful in the treatment of fungal infections in animals, including humans.

EP 0 149 197 discloses pharmaceutical compositions which comprise inclusion compounds of drugs, which are instable or only sparingly soluble in water, with partially etherified beta-cyclodextrin derivatives having hydroxyalkyl and optionally additional alkyl groups.

Accordingly there is a need for formulations of voriconazole that increase its solubility and aqueous stability.

### Summary of the Invention

This invention in general relates to pharmaceutical compositions comprising voriconazole, as well as processes for the preparation of such compositions.

In one aspect, the present invention is directed to a process for preparing a pharmaceutical composition comprising the steps
(i) preparing a solution of voriconazole in N-methylpyrrolidone with a concentration of at least 25 mg voriconazole per ml of N-methylpyrrolidone; and
(ii) mixing the solution obtained in step (i) with a composition containing water in an amount sufficient to obtain an aqueous solution containing N-methylpyrrolidone and voriconazole.

The present invention also provides a process for increasing the solubility of voriconazole in aqueous solutions comprising the steps
(i) preparing a solution of voriconazole in N-methylpyrrolidone with a concentration of at least **25** mg voriconazole per ml of N-methylpyrrolidone; and
(ii) mixing the solution obtained in step (i) with a composition containing water in an amount sufficient to obtain an aqueous solution containing N-methylpyrrolidone and voriconazole.

Preferably, the amount of the composition containing water added in step (ii) is sufficient to obtain an aqueous solution containing N-methylpyrrolidone and voriconazole with from 10 to 50 vol.-% water.

According to another embodiment, the concentration of voriconazole in N-methylpyrrolidone is in the range of from 25 mg/ml to 200 mg/ml.

Step (i) or step (ii) or step (i) and step (ii) are preferably carried out at room temperature.

The present invention also provides the use of N-methylpyrrolidone to improve the solubility of voriconazole in aqueous solutions.

The present invention also provides a pharmaceutical composition comprising
(a) a solution of voriconazole in N-methylpyrrolidone with a concentration of at least 25 mg voriconazole per ml of N-methylpyrrolidone.

Preferably, the pharmaceutical composition comprises from 20 to 99.99 vol.-% of the solution of voriconazole in N-methylpyrrolidone, based on the total amount of the pharmaceutical composition.

According to another embodiment the present invention also provides a composition, wherein the concentration of voriconazole in N-methylpyrrolidone is in the range of from 25 mg/ml to 200 mg/ml.

Preferably, the pharmaceutical composition additionally comprises
(b) 0.01 to 50 vol.-% of water, based on the total amount of the pharmaceutical composition;
   and/or
(c) 0 to 50 vol.-% of additional compounds, based on the total amount of the pharmaceutical composition.

According to another embodiment the present invention also provides a pharmaceutical composition obtainable by a process as described above.

Preferably, the pharmaceutical composition comprises from 20 to 99.99 vol.-% of the solution of voriconazole in N-methylpyrrolidone, based on the total amount of the pharmaceutical composition.

In particular, the concentration of voriconazole in N-methylpyrrolidone is in the range of from 25 mg/ml to 200 mg/ml.

According to another embodiment, the pharmaceutical composition the formulation additionally comprises
(b) 0.01 to 50 vol.-% of water, based on the total amount of the pharmaceutical composition;
   and/or
(c) 0 to 50 vol.-% of additional compounds, based on the total amount of the pharmaceutical composition.

According to another embodiment the present invention also provides a method of treating a fungal infection comprising administering to a patient in need of such treating a therapeutically effective amount of the pharmaceutical composition according to the invention or a pharmaceutical composition obtainable according to a process according to the invention and at least one pharmaceutically acceptable excipient.

According to another embodiment the present invention also provides a pharmaceutical formulation comprising the pharmaceutical composition according to the invention or a composition obtainable according to a process according to the invention and at least one pharmaceutically acceptable excipient.

Preferably, the formulation is used for treating a fungal infection in a mammal, in particular in man.

According to another embodiment the present invention also provides the use of a therapeutically effective amount of the pharmaceutical formulation according to the invention or a pharmaceutical composition obtainable according to a process according to the invention and at least one pharmaceutically acceptable excipient for the preparation of a medicament for treating a fungal infection, preferably wherein said medicament is to be administered to a patient in need thereof.

### Description of the Invention

This invention in general relates to pharmaceutical compositions comprising voriconazole, as well as processes for the preparation of such compositions.

In particular, the present invention is directed to a process for preparing a pharmaceutical composition comprising the steps
(i) preparing a solution of voriconazole in N-methylpyrrolidone with a concentration of at least 25 mg voriconazole per ml of N-methylpyrrolidone; and
(ii) mixing the solution obtained in step (i) with a composition containing water in an amount sufficient to obtain an aqueous solution containing N-methylpyrrolidone and voriconazole.

In the context of the present invention, a "solution of voriconazole in N-methylpyrrolidone with a concentration of at least 25 mg voriconazole per ml of N-methylpyrrolidone" is a solution of voricionazole in N-methylpyrrolidone which can also contain further compounds as long as the concentration of voriconazole in N-methylpyrrolidone is given.

According to the present invention, the solution of voriconazole in N-methylpyrrolidone with a concentration of at least 25 mg voriconazole per ml of N-methylpyrrolidone can be prepared according to any method known to the person skilled in the art. The solution can for example be prepared by adding voriconazole to N-methylpyrrolidone.

It has been found that voriconazole has an extremely high solubility in N-methylpyrrolidone. This can also be used to improve the solubility of voriconazole in aqueous solutions. It has been found that aqueous solutions comprising voriconazole and N-methylpyrrolidone can be prepared with high concentrations of voriconazole according to the process of the present invention.

The present invention also provides a process for increasing the solubility of voriconazole in aqueous solutions comprising the steps
(i) preparing a solution of voriconazole in N-methylpyrrolidone with a concentration of at least 25 mg voriconazole per ml of N-methylpyrrolidone; and
(ii) mixing the solution obtained in step (i) with a composition containing water in an amount sufficient to obtain an aqueous solution containing N-methylpyrrolidone and voriconazole.
   In the context of the present invention, an aqueous solution is a solution which comprises water but can also comprise other solvents, in particular solvents which are miscible with water. The composition containing water can also comprise other compounds such as salts or buffers, preferably compounds which are frequently used in pharmaceutical compositions, such as pharmaceutical acceptable salts.

According to the present invention, the aqueous solution containing N-methylpyrrolidone and voriconazole is preferably optically clear.

According to the present invention, the composition containing water is added to the solution formed in step (i) in an amount sufficient to form an aqueous solution containing N-methylpyrrolidone and voriconazole which is preferably optically clear.

According to the present invention, the composition containing water is preferably added in an amount sufficient to obtain an aqueous solution containing N-methylpyrrolidone and voriconazole with from about 0.01 to about 50 vol.-% water, based on the total amount of the pharmaceutical composition, more preferably from about 10 to about 45 vol.-%, in particular from about 15 to about 40 vol.-%, for example from about 20 to about 35 vol.-% or from about 25 to about 30 vol.-%.

Preferably, the amount of the composition containing water added in step (ii) is sufficient to obtain an aqueous solution containing N-methylpyrrolidone and voriconazole with from about 10 to about 50 vol.-% water.

Therefore, the present invention also provides a process for preparing a pharmaceutical composition as described above, wherein the amount of the composition containing water added in step (ii) is sufficient to obtain an aqueous solution containing N-methylpyrrolidone and voriconazole with from 10 to 50 vol.-% water.

According to the present invention, voriconazole is dissolved in N-methylpyrrolidone to give a solution of voriconazole in N-methylpyrrolidone with a concentration of at least 25 mg voriconazole, preferably 30 mg, more preferably 35 mg, in particular 40 mg, especially preferred 45 mg, more preferred 50 mg voriconazole per ml of N-methylpyrrolidone. Preferably, the concentration of voriconazole in N-methylpyrrolidone is in the range of from about 25 mg/ml to about 200 mg/ml, in particular from about 30 mg/ml to about 175 mg/ml, for example from about 35 mg/ml to about 150 mg/ml, preferably 40 mg/ml to about 125 mg/ml, more preferably 45 mg/ml to about 100 mg/ml, in particular 50 mg/ml to about 75 mg/ml.

Therefore, the present invention also provides a process as described above, wherein the concentration of voriconazole in N-methylpyrrolidone is in the range of from 25 mg/ml to 200 mg/ml.

According to the present invention, other compounds can be added to the composition obtained in step (ii). According to the present invention, other compounds are preferably added in an amount sufficient to obtain an aqueous solution containing N-methylpyrrolidone and voriconazole with from about 0 to about 50 vol.-% of additional compounds, based on the total amount of the pharmaceutical composition, more preferably from about 10 to about 45 vol.-%, in particular from about 15 to about 40 vol.-%, for example from about 20 to about 35 vol.-% or from about 25 to about 30 vol.-%.

According to the present invention, steps (i) and (ii) can be carried out at any suitable temperature, for example at room temperature or at elevated temperature.

Step (i) or step (ii) or step (i) and step (ii) are preferably carried out at room temperature.

Therefore, the present invention also provides a process for preparing a pharmaceutical composition as described above, wherein step (i) or step (ii) or step (i) and step (ii) are carried out at room temperature.

According to the present invention, step (ii) can be carried out directly after step (i). It is also possible that between steps (i) and (ii) further steps are carried out in the context of the present invention.

According to one embodiment of the present invention, steps (i) and (ii) can be carrid out with a time gap. According to the present invention, a stable solution is obtained in step (i) which can be stored. Step (ii) can therefore be carried out after storage of the solution obtained in step (i) prior to the use of the pharmaceutical composition according to the present invention.

The process of the present invention can also comprise further steps such as heating or cooling steps.

The process of the present invention can also comprise other steps of treating the composition obtained in step (ii). It is for example possible to add further compounds to the composition obtained in step (ii), such as solvents.

According to the present invention, it is also possible, that in a further step, more water or an aqueous solution is added to the composition obtained in step (ii).

According to the present invention, further compounds can be added to the composition obtained in step (ii).

According to the present invention, voriconazole can be used as such or in form of pharmaceutically acceptable salts, solvates, or derivatives thereof. According to the present invention derivatives include complexes, prodrugs and isotopically-labelled compounds, as well as salts and solvates thereof.

Pharmaceutically acceptable salts of voriconazole include the acid addition and base salts thereof.

Suitable acid addition salts are formed from acids which form non-toxic salts. Examples include the acetate, aspartate, benzoate, besylate, bicarbonate/carbonate, bisulphate/sulphate, borate, camsylate, citrate, edisylate, esylate, formate, fumarate, gluceptate, gluconate, glucuronate, hexafluorophosphate, hibenzate, hydrochloride/chloride, hydrobromide/bromide, hydroiodide/iodide, isethionate, lactate, malate, maleate, malonate, mesylate, methylsulphate, naphthylate, 2-napsylate, nicotinate, nitrate, orotate, oxalate, palmitate, pamoate, phosphate/hydrogen phosphate/dihydrogen phosphate, saccharate, stearate, succinate, tartrate, tosylate and trifluoroacetate salts.

Suitable base salts are formed from bases which form non-toxic salts. Examples include the aluminium, arginine, benzathine, calcium, choline, diethylamine, diolamine, glycine, lysine, magnesium, meglumine, olamine, potassium, sodium, tromethamine and zinc salts.

For a review on suitable salts, see "Handbook of Pharmaceutical Salts: Properties, Selection, and Use" by Stahl and Wermuth (Wiley-VCH, Weinheim, Germany, 2002).

A pharmaceutically acceptable salt of voriconazole may be readily prepared by mixing together solutions of voriconazole and the desired acid or base, as appropriate. The salt may precipitate from solution and be collected by filtration or may be recovered by evaporation of the solvent. The degree of ionisation in the salt may vary from completely ionised to almost non-ionised.

Voriconazole or a pharmaceutically acceptable salt or derivative thereof may exist in both unsolvated and solvated forms. The term 'solvate' is used herein to describe a molecular complex comprising voriconazole, or a pharmaceutically acceptable salt or derivative thereof, and one or more pharmaceutically acceptable solvent molecules, for example, ethanol. The term 'hydrate' is employed when said solvent is water.

All references herein to voriconazole include references to salts thereof, and to solvates of voriconazole or salts thereof.

The compositions of the invention include voriconazole as hereinbefore defined, polymorphs and prodrugs thereof and isotopically-labelled voriconazole or a pharmaceutically acceptable salt or solvate thereof.

As stated, the invention includes all polymorphs and so-called 'prodrugs' of voriconazole. In respect of the latter, certain derivatives of voriconazole which may have little or no pharmacological activity themselves can, when administered into or onto the body, be converted into voriconazole, for example, by hydrolytic cleavage. Such derivatives are referred to as 'prodrugs'.

Prodrugs in accordance with the invention can, for example, be produced by replacing appropriate functionalities present in voriconazole with certain moieties known to those skilled in the art as 'pro-moieties'.

Voriconazole contains an alcohol functionality (-OH), therefore some examples of prodrugs in accordance with the invention may include an ester or ether thereof, for example, by replacement of the hydrogen by phosphorylation to provide (2R,3S)-2-(2,4-Difluorophenyl)-3-(5-fluoro-4-pyrimidinyl)-1-(1H-1,2,4-triazol-1-yl)-2-butyl dihydrogen phosphate as described in WO 97/28169. Further examples of replacement groups in accordance with the foregoing examples and examples of other prodrug types in accordance with the invention may be found in the aforementioned references.

The present invention also includes isotopically-labelled voriconazole or a pharmaceutically acceptable salt, solvate or derivative thereof, wherein one or more atoms are replaced by atoms having the same atomic number, but an atomic mass or mass number different from the atomic mass or mass number usually found in nature.

According to the present invention, the solubility of voriconazole in aqueous solution is increased.

Therefore, the present invention also provides the use of N-methylpyrrolidone to improve the solubility of voriconazole in aqueous solutions.

The present invention also provides a pharmaceutical composition comprising
(a) a solution of voriconazole in N-methylpyrrolidone with a concentration of at least 25 mg voriconazole per ml of N-methylpyrrolidone.

Preferably, the pharmaceutical composition comprises from 20 to 99.99 vol.-% of the solution of voriconazole in N-methylpyrrolidone, based on the total amount of the pharmaceutical composition. Preferably, the pharmaceutical composition comprises from about 25 to about 90 vol.-% of the solution of voriconazole in N-methylpyrrolidone, based on the total amount of the pharmaceutical composition, more preferably from about 30 to about 80 vol.-%, in particular form about 40 to about 70 vol.-%.

According to another embodiment the present invention also provides a composition, wherein the concentration of voriconazole in N-methylpyrrolidone is in the range of from 25 mg/ml to 200 mg/ml.

Preferably, the pharmaceutical composition additionally comprises
(b) 0.01 to 50 vol.-% of water, based on the total amount of the pharmaceutical composition;
   and/or
(c) 0 to 50 vol.-% of additional compounds, based on the total amount of the pharmaceutical composition.

With regard to the amounts of water and additional compounds, reference is made to the disclosure regarding the preferred embodiments of the process according to the present invention.

The present invention also relates to the compositions which can be obtained by any process as described above. Therefore, according to another aspect, the present invention also provides a composition comprising water and voriconazole and N-methylpyrrolidone obtainable by a process as described above.

Again, reference is made to preferred embodiments described in the context of the process of the present invention. In particular, reference is made to the explanation regarding voriconazole.

Preferably, the pharmaceutical composition comprises from 20 to 99.99 vol.-% of the solution of voriconazole in N-methylpyrrolidone, based on the total amount of the pharmaceutical composition.

In particular, the concentration of voriconazole in N-methylpyrrolidone is in the range of from 25 mg/ml to 200 mg/ml.

According to another embodiment, the pharmaceutical composition the formulation additionally comprises
(b) 0.01 to 50 vol.-% of water, based on the total amount of the pharmaceutical composition;
   and/or
(c) 0 to 50 vol.-% of additional compounds, based on the total amount of the pharmaceutical composition.

The compositions obtained in a process according to the present invention or the compositions of the present invention can be used as antifungal agents. The compositions according to the present invention are suitable for the use in pharmaceutical compositions.

In a further aspect the invention therefore provides a method of treating a fungal infection comprising administering to a patient in need of such treating a therapeutically effective amount of the pharmaceutical composition according to the present invention or a pharmaceutical composition obtainable according to a process according to the present invention and at least one pharmaceutically acceptable excipient.

In a further aspect the present invention also provides the use of a therapeutically effective amount of the pharmaceutical composition and/or formulation according to the present invention or a pharmaceutical composition obtainable according to a process according to the present invention and at least one pharmaceutically acceptable excipient for the preparation of a medicament for treating a fungal infection, preferably wherein said medicament is to be administered to a patient in need thereof.

Fungal infections which may be treated by voriconazole have been extensively described in the literature, including EP-A 440 372. For example, they are useful in treating topical fungal infections in man caused by, among other organisms, species of Candida, Trichophyton, Microsporum or Epidermophyton, or in mucosal infections caused by Candida albicans (e.g. thrush and vaginal cadidiasis). They can also be used in the treatment of systemic fungal infections caused by, for example, species of Candida (e.g. Candida albicans), Cryptococcus neoformans, Aspergillus flavus, Aspergillus fumigatus, Coccidioides, Paracoccidioides, Histoplasma or Blastomyces. It will be appreciated that reference to treatment is intended to include prophylaxis as well as the alleviation of established symptoms.

Likewise, EP-A 440 372 also describes a suitable dose for the parenteral administration of voriconazole. A typical dose is between 0.1 to 25 mg/kg, such as 0.5 to 20 mg/kg, for example 1 to 10 mg/kg, administered twice a day. It will be appreciated that the precise therapeutic dose of voriconazole will depend on the age and condition of the patient and the nature of the condition to be treated and will be at the ultimate discretion of the attendant physician.

According to a further aspect, the present invention also provides a pharmaceutical formulation comprising the pharmaceutical composition according to the present invention or a composition obtainable according to a process according to the present invention and at least one pharmaceutically acceptable excipient.

Preferably, the pharmaceutical formulation is a pharmaceutical formulation for treating a fungal infection in a mammal, in particular in man.

Formulations of the present invention suitable for parenteral administration need very few constituents, but it is generally preferred that an injectable solution is made up with saline to provide a solution which is iso-osmotic with blood. These compositions can be sterilised after preparation. They can be provided in any suitable form and in any suitable containers appropriate to maintaining sterility.

The present invention is suitable for pharmaceutical use. The formulations can be administered by various routes. Preferred routes of administration are parenteral and oral.

Modes of parenteral administration include intravenous, intra-arterial, intrathecal, intraperitoneal, intraocular, intra-articular, intramuscular, subcutaneous injection, and the like. The present invention may also be administered via other routes that include oral, buccal, periodontal, rectal, nasal, pulmonary, transdermal, or topical. In an embodiment of the present invention, the aqueous medium of the composition is removed to form dry particles. The method to remove the aqueous medium can be any method known in the art. One example is evaporation. Another example is freeze drying or lyophilization. The dry particles may then be formulated into any acceptable physical form including, but is not limited to, solutions, tablets, capsules, suspensions, creams, lotions, emulsions, aerosols, powders, incorporation into reservoir or matrix devices for sustained release (such as implants or transdermal patches), and the like. Administration routes of these pharmaceutical forms include, but are not limited to parenteral, oral, buccal, periodontal, rectal, nasal, pulmonary, transdermal and topical. Furthermore, the active pharmaceutical agent may be delivered using controlled or sustained release formulations, incorporation into delivery.

Preferably, the formulations is for parenteral administration, for example, i.v. administration.

Not withstanding above concentration range disclosures of an intermediate which is a solution of voriconazole in N-methylpyrrolidone, generally, in aqueous intravenous and intramuscular formulations according to the invention, the voriconazole will be present at a concentration of from 5 mg/ml to 50 mg/ml, for example 10 mg/ml to 30 mg/ml. The formulations may in some instances be lyophilised (freeze dried) for storage prior to use, and made up with water when required.

For human use, voriconazole and its salts can be administered alone, but will generally be administered in admixture with a pharmaceutical carrier selected with regard to the intended route of administration and standard pharmaceutical practice. For example, they can be administered orally in the form of tablets containing such excipients as starch or lactose, or in capsules or ovules either alone or in admixture with excipients, or in the form of elixirs, solutions or suspensions containing flavouring or colouring agents. They can be injected parenterally, for example, intravenously, intramuscularly or subcutaneously. For parenteral administration, they are best used in the form of a sterile aqueous solution which may contain other substances, for example, enough salts or glucose to make the solution isotonic with blood.

For oral and parenteral administration to human patients, the daily dosage level of a formulation comprising voriconazole and its salts will be from 0.01 to 20 mg/kg (in single or divided doses) when administered by either the oral or parenteral route. Thus tablets or capsules of the compounds will contain from 5 mg to 0.5 g of active compound for administration singly or two or more at a time, as appropriate. The above dosages are exemplary of the average case; there can, of course, be individual instances where higher or lower dosage ranges are merited, and such are within the scope of this invention.

Alternatively, the voriconazole can be administered in the form of a suppository or pessary, or they may be applied topically in the form of a lotion, solution, cream, ointment or dusting powder. For example, it can be incorporated into a cream consisting of an aqueous emulsion of polyethylene glycols or liquid paraffin; or it can be incorporated, at a concentration between 1 and 10%, into an ointment consisting of a white wax or white soft paraffin base together with such stabilizers and preservatives as may be required.

It is generally preferred that compositions of the present formulations are provided in a form suitable for direct injection. It will be readily appreciated by those skilled in the art how to administer compositions of the present invention to a human or animal.

Preferably, the formulations of the present invention are provided in a syringe with two separate barrels. In this case, one barrel contains for example the composition obtained in the process according to the present invention or the composition obtained in step (i) of the process according to the present invention and the second barrel can contain for example water or an aqueous solution containing further compounds and/or pharmaceutical acceptable salts. According to this embodiment, step (ii) of the process according to the present invention can be carried out when using the syringe with two separate barrels.

Parenteral formulations are typically aqueous solutions which may contain excipients such as salts, carbohydrates and buffering agents (preferably to a pH of from 3 to 9), but, for some applications, they may be more suitably formulated as a sterile non-aqueous solution or as a dried form to be used in conjunction with a suitable vehicle such as sterile, pyrogen-free water.

The preparation of parenteral formulations under sterile conditions, for example, by lyophilisation, may readily be accomplished using standard pharmaceutical techniques well known to those skilled in the art.

Formulations for parenteral administration may be formulated to be immediate and/or modified release. Modified release formulations include delayed-, sustained-, pulsed-, controlled-, targeted and programmed release. Thus compositions of the invention may be formulated as a solid, semi-solid, or thixotropic liquid for administration as an implanted depot providing modified release of the active compound. Examples of such formulations include drug-coated stents and PGLA microspheres.

The formulations of the invention may also be administered topically to the skin or mucosa, that is, dermally or transdermally. Typical formulations for this purpose include gels, hydrogels, lotions, solutions, creams, ointments, dressings, foams, films, skin patches, wafers, implants, sponges, fibres, bandages and microemulsions. Liposomes may also be used. Typical carriers include alcohol, water, mineral oil, liquid petrolatum, white petrolatum, glycerin, polyethylene glycol and propylene glycol. Penetration enhancers may be incorporated.

Other means of topical administration include delivery by electroporation, iontophoresis, phonophoresis, sonophoresis and microneedle or needle-free (e.g. Powderject(TM), Bioject(TM), etc.) injection.

Formulations for topical administration may be formulated to be immediate and/or modified release. Modified release formulations include delayed-, sustained-, pulsed-, controlled-, targeted and programmed release.

The formulations of the invention can also be administered intranasally or by inhalation, typically as an aerosol spray from a pressurised container, pump, spray, atomiser (preferably an atomiser using electrohydrodynamics to produce a fine mist), or nebuliser, with or without the use of a suitable propellant, such as 1,1,1,2-tetrafluoroethane or 1,1,1,2,3,3,3-heptafluoropropane.

The pressurised container, pump, spray, atomizer, or nebuliser contains a solution of the compositions of the invention comprising, for example, ethanol (optionally, aqueous ethanol) or a suitable alternative agent for dispersing, solubilising, or extending release of the active, the propellant(s) as solvent and an optional surfactant, such as sorbitan trioleate, oleic acid, or an oligolactic acid.

A suitable solution formulation for use in an atomiser using electrohydrodynamics to produce a fine mist may contain from 1 µg to 20 mg of voriconazole or pharmaceutically acceptable salts, solvates, or derivatives thereof per actuation and the actuation volume may vary from 1 µl to 100 µl. A typical formulation may comprise a formulation of the invention, propylene glycol, sterile water, ethanol and sodium chloride. Alternative solvents which may be used instead of propylene glycol include glycerol and polyethylene glycol.

Suitable flavours, such as menthol and levomenthol, or sweeteners, such as saccharin or saccharin sodium, may be added to those formulations of the invention intended for inhaled/intranasal administration.

Formulations for inhaled/intranasal administration may be formulated to be immediate and/or modified release using, for example, poly-DL-lactic-coglycolic acid (PGLA). Modified release formulations include delayed-, sustained-, pulsed-, controlled-, targeted and programmed release.

In the case of aerosols, the dosage unit is determined by means of a valve which delivers a metered amount. Units in accordance with the invention are typically arranged to administer a metered dose or "puff" containing from 1 µg to 10 mg of voriconazole or pharmaceutically acceptable salts, solvates, or derivatives thereof. The overall daily dose will typically be in the range 1 µg to 200 mg which may be administered in a single dose or, more usually, as divided doses throughout the day.

The formulations of the invention may also be administered directly to the eye or ear, typically in the form of drops of solution in isotonic, pH-adjusted, sterile saline. Other formulations suitable for ocular and aural administration include ointments, biodegradable (e.g. absorbable gel sponges, collagen) and non-biodegradable (e.g. silicone) implants, wafers, lenses and particulate or vesicular systems, such as niosomes or liposomes. A polymer such as crossed-linked polyacrylic acid, polyvinylalcohol, hyaluronic acid, a cellulosic polymer, for example, hydroxypropylmethylcellulose, hydroxyethylcellulose, or methyl cellulose, or a heteropolysaccharide polymer, for example, gelan gum, may be incorporated together with a preservative, such as benzalkonium chloride. Such formulations may also be delivered by iontophoresis.

Formulations for ocular/aural administration may be formulated to be immediate and/or modified release. Modified release formulations include delayed-, sustained-, pulsed-, controlled-, targeted, or programmed release.

The following examples illustrate the process of the present invention and are not intended to limit the scope of the invention set forth in the claims appended thereto.

### Examples

Voriconazole is synthesized by method known in the art, e.g.:according to EP-A 440 372, while N-Methylpyrrolidone (known under name Pharmasolve^{®}) is obtained from ISP Technologies Inc., Texas, USA.

### Example 1:

0.210 g Voriconazole are dissolved in 50 mL N-Methylpyrrolidone (NMP) using a magnetic stirrer. Further aliquots of 0.305 g, 0.519 g and 1.009 Voriconazole are added to NMP-solution using magnet stirring. Solution I is obtained, composed of 2.017 g Voriconazole in 50 mL NMP.

Solution I is diluted with water according to following scheme:

| **solution** | **Volume solution I** | **Volume water** | **Total Volume** | **amount of voriconazole** | **Concentration of voriconazole** |
|---|---|---|---|---|---|
| I | 50 mL | 0 mL | 50.0 mL | 2.017 g | 40.34 mg/mL |
| IIa | 12.5 mL | 12.5 mL | 25.0 mL | 0.50425 g | 20.17 mg/mL |
| IIb | 12.5 mL | 25.0 mL | 37.5 mL | 0.50425 g | 13.45 mg/mL |
| IIc | 12.5 mL | 37.5 mL | 50.0 mL | 0.50425 g | 10.085 mg/mL |

Clarity of solution is analysed by optical inspection. A solution containing 50 % of water is still clear after stirring overnight. Containing 66 % of water, a slightly turbid solution is obtained. Floculation of Vvoriconazole is visible. Containing 75 % of water, a turbid solution is obtained. Flocculation of Voriconazole is visible.

| **solution** | **c_{Voriconazole}** | **c_{NMP}** | **c_{water}** | **clarity of solution** |
|---|---|---|---|---|
| I | 40.34 mg/mL | 100 % | --- | clear |
| IIa | 20.17 mg/mL | 50 % | 50 % | clear |
| IIb | 13.45 mg/mL | 33 % | 67 % | turbid |
| IIc | 10.085 mg/mL | 25 % | 75 % | turbid |

### Example 2:

0.45 - 0.55 g Voriconazole are dissolved in NMP according to example 1. After obtaining a clear solution, solutions are made up with water to 20.0 mL.

The following solutions are prepared:

| **solution** | **m_{Voriconazole} [g]** | **V_{NMP} [mL]** | **V_{Water} [mL]** |
|---|---|---|---|
| IIIa | 0.52 | 20 | 0 |
| IIIb | 0.51 | 16 | 4 |
| IIIc | 0.52 | 12 | 8 |
| IIId | 0.50 | 8 | 12 |
| IIIe | 0.51 | 4 | 16 |
| IIIf | 0.52 | 0 | 20 |

Clarity of solution is analysed by optical inspection. The following results are obtained:

| **solution** | **c_{Voriconazole}** | **c_{NMP}** | **c_{water}** | **clarity of solution** |
|---|---|---|---|---|
| IIIa | 25 mg/mL | 100 % | 0 % | clear |
| IIIb | 25 mg/mL | 80 % | 20 % | clear |
| IIIc | 25 mg/mL | 60 % | 40 % | clear |
| IIIc | 25 mg/mL | 40 % | 60 % | turbid |
| IIIc | 25 mg/mL | 20 % | 80 % | turbid |
| IIIc | 25 mg/mL | 0 % | 100 % | turbid |

Calculated based on a formulation with a volume of 20 mL this would result in formulations composed of:

| **solution** | **m_{Voriconazole} [g]** | **V_{NMP} [mL]** | **V_{water} [mL]** | **clarity of solution** |
|---|---|---|---|---|
| IVa | 0.50 | 20 | 0 | clear |
| IVb | 0.50 | 16 | 4 | clear |
| IVc | 0.50 | 12 | 8 | clear |
| IVc | 0.50 | 8 | 12 | turbid |
| IVc | 0.50 | 4 | 16 | turbid |
| IVc | 0.50 | 0 | 20 | turbid |

Therefore, in 20 mL solution, 0.5 g Voriconazole can be dissolved with water / NMP mixtures.

## Claims

1. A process for preparing a pharmaceutical composition comprising the steps
(i) preparing a solution of voriconazole in N-methylpyrrolidone with a concentration of at least 25 mg voriconazole per ml of N-methylpyrrolidone; and
(ii) mixing the solution obtained in step (i) with a composition containing water in an amount sufficient to obtain an aqueous solution containing N-methylpyrrolidone and voriconazole.

2. A process for increasing the solubility of voriconazole in aqueous solutions comprising the steps
(i) preparing a solution of voriconazole in N-methylpyrrolidone with a concentration of at least 25 mg voriconazole per ml of N-methylpyrrolidone; and
(ii) mixing the solution obtained in step (i) with a composition containing water in an amount sufficient to obtain an aqueous solution containing N-methylpyrrolidone and voriconazole.

3. The process according to any of claims 1 or 2, wherein the amount of the composition containing water added in step (ii) is sufficient to obtain an aqueous solution containing N-methylpyrrolidone and voriconazole with from 10 to 50 vol.-% water.

4. The process according to any of claims 1 to 3, wherein the concentration of voriconazole in N-methylpyrrolidone is in the range of from 25 mg/ml to 200 mg/ml.

5. The process according to any of claims 1 to 4, wherein step (i) or step (ii) or step (i) and step (ii) are carried out at room temperature.

6. Use of N-methylpyrrolidone to improve the solubility of voriconazole in aqueous solutions.

7. A pharmaceutical composition comprising
(a) a solution of voriconazole in N-methylpyrrolidone with a concentration of at least 25 mg voriconazole per ml of N-methylpyrrolidone.

8. The pharmaceutical composition of claim 7, wherein the pharmaceutical composition comprises from 20 to 99.99 vol.-% of the solution of voriconazole in N-methylpyrrolidone, based on the total amount of the pharmaceutical composition.

9. The pharmaceutical composition of claim 7 or 8, wherein the concentration of voriconazole in N-methylpyrrolidone is in the range of from 25 mg/ml to 200 mg/ml.

10. The pharmaceutical composition according to any of claims 7 to 9, wherein the pharmaceutical composition additionally comprises
(b) 0.01 to 50 vol.-% of water, based on the total amount of the pharmaceutical composition;
and/or
(c) 0 to 50 vol.-% of additional compounds, based on the total amount of the pharmaceutical composition.

11. A pharmaceutical composition obtainable by a process according to any of claims 1 to 5 comprising water and voriconazole in N-methylpyrrolidone with a concentration of at least 25 mg voriconazole per ml of N-methylpyrrolidone.

12. The pharmaceutical composition of claim 11, wherein the pharmaceutical composition comprises from 20 to 99.99 vol.-% of the solution of voriconazole in N-methylpyrrolidone, based on the total amount of the pharmaceutical composition.

13. The composition of claim 11 or 12, wherein the concentration of voriconazole in N-methylpyrrolidone is in the range of from 25 mg/ml to 200 mg/ml.

14. The pharmaceutical composition according to any of claims 11 to 13, wherein the pharmaceutical composition additionally comprises
(b) 0.01 to 50 vol.-% of water, based on the total amount of the pharmaceutical composition;
and/or
(c) 0 to 50 vol.-% of additional compounds, based on the total amount of the pharmaceutical composition.

15. A method of treating a fungal infection comprising administering to a patient in need of such treating a therapeutically effective amount of the pharmaceutical composition according to any of claims 7 to 14 or a pharmaceutical composition obtainable according to a process according to any of claims 1 to 5 and at least one pharmaceutically acceptable excipient.

16. A pharmaceutical formulation comprising the pharmaceutical composition according to any of claims 7 to 14 or a pharmaceutical composition obtainable according to a process according to any of claims 1 to 5 and at least one pharmaceutically acceptable excipient.

17. A formulation according to claim 16 for treating a fungal infection in a mammal, in particular in man.

18. Use of a therapeutically effective amount of the pharmaceutical formulation according to any of claims 16 or 17 or a pharmaceutical composition obtainable according to a process according to any of claims 1 to 5 and at least one pharmaceutically acceptable excipient for the preparation of a medicament for treating a fungal infection, preferably wherein said medicament is to be administered to a patient in need thereof.
